# EUROPEAN PATENT SPECIFICATION

(11) **EP 1 212 078 B1**
(45) Date of publication and mention of the grant of the patent: **22.06.2005**
(21) Application number: 00965952.5
(22) Date of filing: 08.09.2000
(51) Int. Cl.: A61K 38/17, A61K 38/52, G01N 33/50, A61P 9/00

(54) **ANT-1 AS DRUG TARGET**
ANT-1 ALS THERAPEUTISCHES ZIELMOLEKÜL
ANT-1 COMME CIBLE DE PRINCIPE ACTIF

(30) Priority: 10.09.1999 US 153040 P
(43) Date of publication of application: 12.06.2002
(73) Proprietor: Max-Planck-Gesellschaft zur Förderung der Wissenschaften e.V., 80539 München (DE)
(72) Inventor: GRIMM, Stefan, 81241 München (DE); BAUER, Manuel, 80686 München (DE); SCHUBERT, Alexis, 80333 München (DE); CRAMER, Ursula, 81241 München (DE)
(74) Representative: Weiss, Wolfgang, Dipl.-Chem. Dr.
(86) International application number: PCT/EP2000/008812
(87) International publication number: WO 2001/019384

(56) References cited:
- EP-A- 1 090 924
- WO-A-00/26370
- DOERNER A ET AL: "Adenine nucleotide translocator in dilated cardiomyopathy: Pathophysiological alterations in expression and function." MOLECULAR AND CELLULAR BIOCHEMISTRY, vol. 174, no. 1-2, 1997, pages 261-269, XP000990253
- SYLVEN C ET AL: "Ventricular adenine nucleotide translocator mRNA is upregulated in dilate cardiomyopathy." CARDIOVASCULAR RESEARCH, vol. 27, no. 7, July 1993 (1993-07), pages 1295-1299, XP000990203
- HALESTRAP A P ET AL: "Elucidating the molecular mechanism of the permeability transition pore and its role in reperfusion injury of the heart." BIOCHIMICA ET BIOPHYSICA ACTA, vol. 1366, no. 1-2, 10 August 1998 (1998-08-10), pages 79-94, XP000982881
- KHASPEKOV L ET AL: "Cyclosporin A and its nonimmunosuppressive analogue N-Me-Val-4-cyclosporin A mitigate glucose/oxygen deprivation-induced damage to rat cultured hippocampal neurons." EUROPEAN JOURNAL OF NEUROSCIENCE, vol. 11, no. 9, 1999, pages 3194-3198, XP000982879
- BAUER M K A ET AL: "Adenine nucleotide translocase-1, a component of the permeability transition pore, can dominantly induce apoptosis." JOURNAL OF CELL BIOLOGY, vol. 147, no. 7, 27 December 1999 (1999-12-27), pages 1493-1501, XP000990027

## Description

The present invention refers to the use of adenine nucleotide translocase-1 (ANT-1), a central component of the permeability transition pore in mitochondria, as a drug target, particularly for the treatment of dilated cardiomyopathy. Further, an assay for the detection of pharmacologically active substances is disclosed which inhibit ANT-1 activity. In addition, ANT-1 expression is claimed as a diagnostic marker for dilated cardiomyopathy.

Apoptosis is a form of cell death that plays a role in development, tissue homeostasis and disease (White, 1996). Its induction must be tightly regulated. Otherwise, serious consequences may follow: A hyperactive apoptosis induction might lead to degenerative diseases like Alzheimer's disease (Loo et al., 1993); a reduced activity can contribute to the multistep process of tumorigenesis, since tumor cells are exposed to multiple proapoptotic stimuli (McGill, 1997). The induction of apoptosis is therefore governed by an elaborate array of checks and balances in the cell. Eventually, a family of cysteine proteases, the so-called caspases, is activated (Salvesen and Dixit, 1997). These enzymes can cleave specific substrates in the cell which leads to the typical apoptotic phenotype and the self-destruction of the cell.

The first indication that mitochondria play a role in apoptosis induction was the observation that an in vitro system for apoptosis induction required the presence of mitochondria (Newmeyer et al., 1994). The permeability transition pore (PT) complex (Zoratti and Szabo, 1995), a protein aggregate that resides in contact sites of the inner and outer mitochondrial membrane, was subsequently identified as being responsible for apoptosis induction (Petit et al., 1995; Zamzami et al., 1995b). Multiple pharmacological stimuli have been shown to activate this complex by as yet unknown means (Fulda et al., 1998; Petit et al., 1995; Zamzami et al., 1998). The activated complex leads to the collapse of the potential over the inner mitochondrial membrane (Δψₘ), swelling of mitochondria and the generation of oxygen radicals (Vander Heiden et al., 1997; Zamzami et al., 1995a). The subsequent release of apoptogenic caspases (Susin et al., 1999a) and a putative oxidoreductase (Susin et al., 1999b) aids in apoptosis induction.

Many genes involved in apoptosis have the dominant capacity to induce cell death upon overexpression. This feature is even conserved across species (McCarthy and Dixit, 1998). It might be explained by the fact that the overexpressed proteins engage in protein-protein contacts and can thereby create the signal for apoptosis (Yang et al., 1998). Consequently, we have recently developed a screen for dominant, apoptosis-inducing genes (Grimm and Leder, 1997). The screen is based on the iterative transfection of small plasmid pools of a normalized cDNA library into mammalian cells and the morphological determination of apoptosis induction.

Here we describe the isolation of adenine nucleotide translocase-1 (ANT-1), a central component of the permeability transition pore, using such a screen. ANT-1 has recently been shown to be required for apoptosis mediated by Bax, another component of the PT complex (Marzo et al., 1998a). Conversely, ANT-1 can induce apoptosis in a Bax-dependent manner. For this, ANT-1 must be activated pharmacologically by the chemical atractyloside which arrests ANT-1 in a specific conformation and causes PT pore opening (Marzo et al., 1998a). In our experiments with ANT-1, however, such a secondary signal is not required for apoptosis induction. ANT-1's apoptosis activity does not seem to depend on its known function for ADP/ATP exchange because several transport inactive mutants could still lead to cell death. Surprisingly, we found that a very homologous protein, the ANT-2 transporter, was inactive for apoptosis induction. Furthermore, ANT-1 could not elicit a form of cell death in yeast. This is in contrast to Bax that directly interacts with ANT-1 and can dominantly induce cell death in yeast. This result suggests that ANT-1-mediated apoptosis induction depends on protein-protein interactions that are specific for mammalian cells and is independent of Bax-mediated apoptosis. Since ANT-1 is activated by overexpression for apoptosis induction, it is interesting to note that this gene is already highly expressed in mitochondria. This suggests that in a normal cell it must be kept inactive by other proteins of the PT pore which implies important stoichiometric correlations between the various components of this complex. Consistent with this, we found that cyclophilin D, another component of the PT pore can repress ANT-1-induced apoptosis. Furthermore, our data help to explain the observed apoptosis induction in dilated cardiomyopathy (DCM), a degenerative disease of the heart muscle which is marked by a dramatic increase of the expression level of ANT-1 and by excessive apoptosis induction.

These findings are up to now the most direct genetic evidence that the PT pore can signal apoptosis and may lead to the molecular elucidation of how this complex can be activated for apoptosis induction.

A first aspect of the present invention is the use of an effective amount of a substance capable of inhibiting the activity of adenine nucleotide translocase-1 (ANT-1), particularly the apoptosis-inducing activity of ANT-1, for the inhibition of apoptosis in an isolated cell.

The cell is preferably a vertebrate cell, more preferably a mammalian cell and, still more preferably, a human cell, e.g. a human myocyte.

The inhibition may effect an apoptosis-inducing signal transduction pathway, said pathway being activated by ANT-1. The activity of ANT-1 may be inhibited on the nucleic acid level and/or on the protein level. inhibition on the nucleic acid level may be effected by reducing ANT-1 gene expression, e.g. by using antisense-oligonucleotides which may inhibit the ANT-1 gene transcription, by using ribozymes capable of cleaving ANT-1 transcripts or by inhibiting the activity of the endogenous promoter, e.g. via ANT-1-specific transcription factors. The reduction of ANT-1 expression may also be achieved by influencing the activity of proteins that act upstream of these transcription factors, e.g. such as protein kinases and phosphatases. Inhibition on the protein level may be effected by anti-ANT-1 antibodies, by ANT-1 regulatory proteins, e.g. by proteins occurring in permeability transition pores such as cyclophilin D or by low molecular weight substances which are capable of inhibiting the apoptotic or proapoptotic activity of ANT-1.

A further subject matter of the present invention is the use of a pharmaceutically effective amount of a substance capable of inhibiting the activity of adenine nucleotide transtocase-1 (ANT-1), particularly the apoptosis-inducing activity of ANT-1 for the manufacture of a medicament for the treatment of degenerative diseases associated with excessive apoptosis. Examples of diseases which may be treated by administering ANT-1 antagonists are dilated cardiomyopathy and other human pathogenic disorders associated with excessive apoptosis. More preferably, the disease is dilated cardiomyopathy.

The ANT-1 antagonist may be administered as a pharmaceutical composition comprising the active agent, optionally together with pharmaceutically acceptable diluents, carriers or adjuvants. The pharmaceutical composition may be suitable for oral, parenteral, e.g. intradermal, intravenous or intramuscular, rectal, nasal and topical applications. The composition may be an injectable solution, ointment, cream or spray. Further, the composition may have retardation properties, e.g. showing a delayed release of the active agent.

The dosage of the active agent depends on the specific compound being administered, the type and the severity of the disease. For example, a dosage from 0.01 mg to 100 mg per day and per kg body weight of the active agent is suitable.

Still a further aspect of the present invention is an in vitro method for identifying substances suitable for apoptosis inhibition, comprising the step of determining the capability of a test substance to inhibit the activity of ANT-1, particularly the apoptosis-inducing activity of ANT-1. Preferably, the capability of a test substance to bind ANT-1 or a domain thereof may be determined. Particularly, the N-terminal domain of ANT-1 comprising amino acids 1-150, preferably amino acids 1-200, is suitable for determining the binding of test substances. Alternatively, the capability of test substances to inhibit the binding of ANT-1 to natural binding partners thereof such as cyclophilin D may be determined. Thus, a substance which binds ANT-1 or a domain thereof or inhibits the binding of ANT-1 to natural binding partners thereof is identified as an apoptosis inhibitor.

The identification of ANT-1 antagonists may be carried out as a high-throughput assay which preferably comprises a parallel determination of at least 24 and, more preferably, at least 96 test compounds. The assay may be carried out as a cell-based assay, wherein a test system is used comprising ANT-1-containing cell fractions or ANT-1-containing whole cells. Alternatively, the assay may be carried out as a molecular-based assay comprising a substantially purified and isolated protein selected from ANT-1 or a domain thereof, particularly an N-terminal domain thereof. The substantially purified ANT-1 protein may be a recombinant ANT-1 protein which may a soluble protein (by deletion of membrane anchor domains) or a membrane-bound protein.

In a cell-based assay the determining step may comprise the measurement of apoptosis induction. Apoptosis induction may be measured by a parameter which is associated with apoptosis such as DNA fragmentation, caspase activation or characteristic alterations in cell morphology or other qualitative and/or quantitative methods of apoptosis measurement known in the field. In molecular-based assay systems the determination may comprise the measurement of protein-protein interactions, e.g. the interaction of ANT-1 with other proteins, or direct measurement of the interaction of test compounds with ANT-1.

As the invention describes upregulated ANT-1 as a drug target in dilated cardiomyopathy or other degenerative diseases, a further aspect of this invention is the examination of the ANT-1 expression level in patients as a diagnostic marker. The ANT-1 expression can be detected and quantified by conventional methods, e.g. on the RNA level by hybridization and/or amplification procedures or on the protein level by antibody and/or activity assays. Thus, the invention relates to an in vitro method for the diagnosis of a degenerative disease or a predispositon therefor comprising detecting the ANT-1 expression in a sample from tissue and/or body fluids of a subject to be tested, wherein elevated ANT-1 expression is indicative for a degenerative disease or a predisposition therefor.

The present invention is further illustrated by the following Figures and Examples.

### Description of Figures

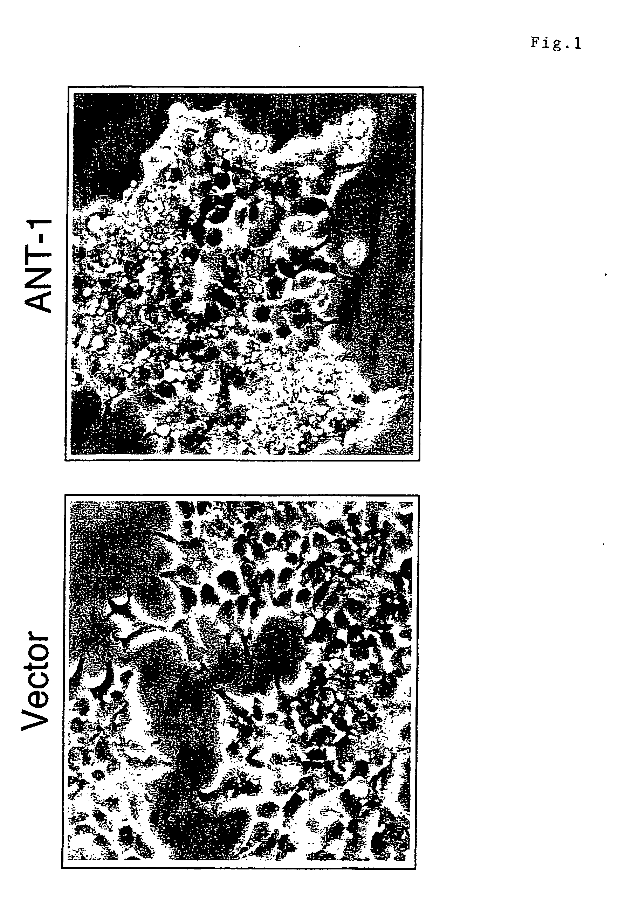

**Fig. 1**. Adenine nucleotide translocase-1 (ANT-1) expression leads to phenotypic apoptosis induction. The empty vector or an expression plasmid for ANT-1 were transiently transfected into 293T cells. After 16 hours, phase contrast pictures were taken at a 200-fold magnification.

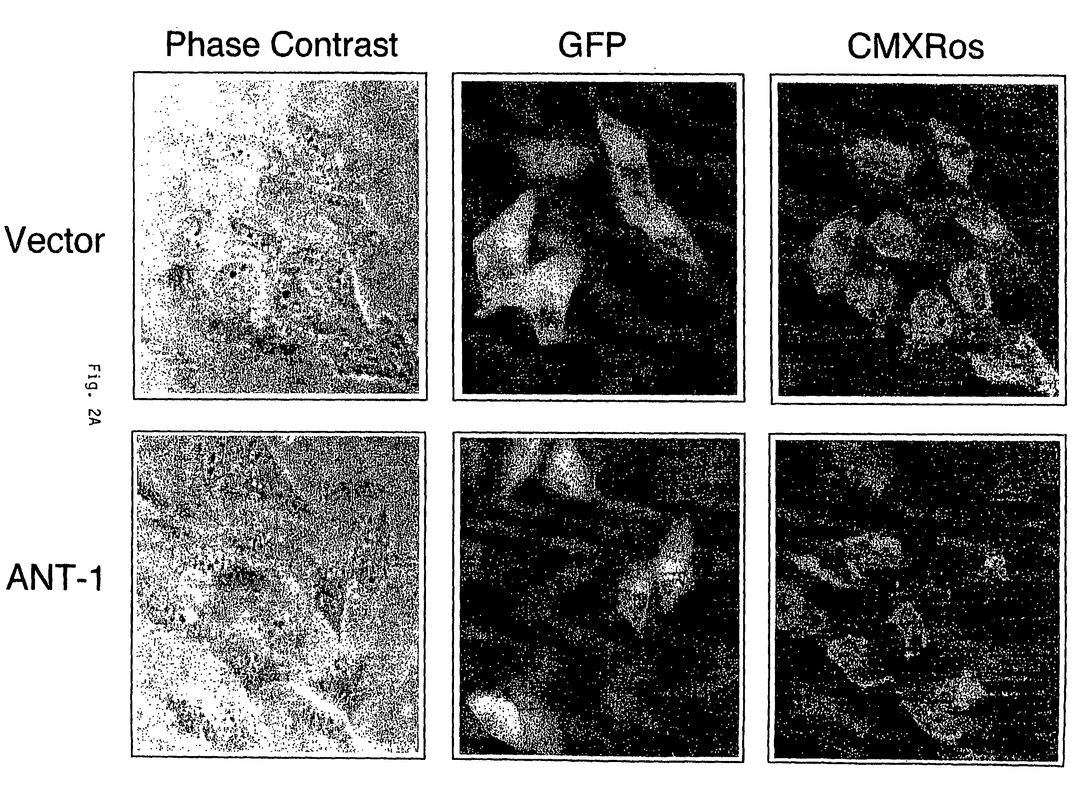

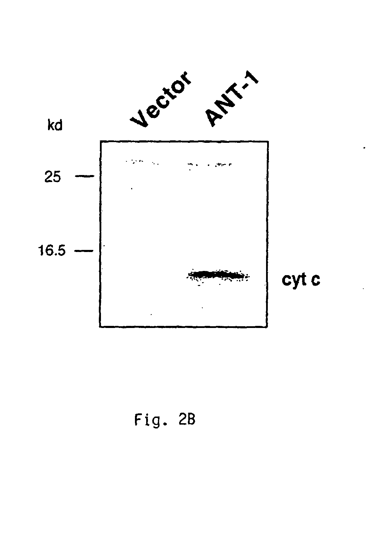

**Fig. 2**. Effect of ANT-1 expression on mitochondria. (**A**) ANT-1 overexpression leads to the collapse of the inner mitochondrial membrane potential. HeLa cells were transfected with an expression vector for GFP (2 µg) together with a control vector ("Vector"; 2 µg) or a plasmid for ANT-1 ("ANT-1"; 2 µg). After 16 hours, the cells were treated with CMXRos which stains mitochondria with an intact membrane potential. Subsequently, phase contrast and fluorescence microscopy pictures for GFP and CMXRos activity were taken. (**B**) ANT-1 induces the release of cytochrome c from mitochondria. 293T cells were transfected with a control vector or an expression vector for ANT-1. Cytoplasmic extracts were prepared and assayed for the presence of cytochrome c by a western blot. Molecular weight standards are given on the left. Equal loading of the gel is indicated by an upper unspecific band with equal intensity.

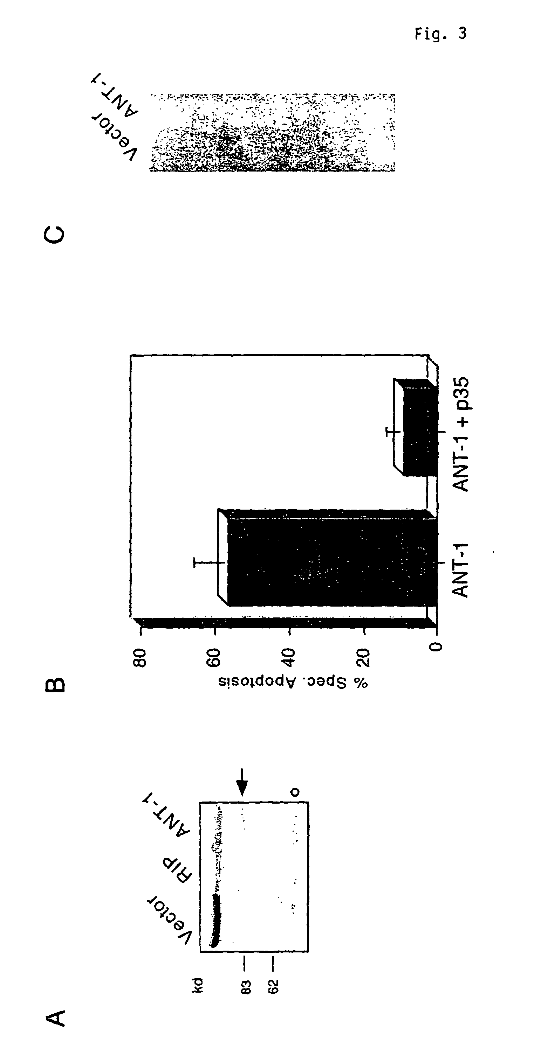

**Fig. 3.** Effect of ANT-1 on protein and DNA degradation. (**A**) ANT-1 leads to the degradation of poly-ADP-ribose polymerase (PARP). 293T cells were transfected with an empty control vector, an expression vector for the "death domain" protein RIP or with ANT-1. 16 hours later, nuclear extracts of the transfected cells were prepared and investigated for the status of PARP in a western blot. The generated PARP fragment is indicated by an arrow. An unspecific signal (o) served as an internal control for equal loading of the gel. (**B**) Inhibition of ANT-1 apoptosis by a specific inhibitor for caspases. ANT- 1 (1 µg) and a control vector (10 µg) or an expression vector (10 µg) for the caspase inhibitor p35 from baculovirus were cotransfected, and the specific apoptosis induction was determined by FACS analysis. The means and the standard deviations are indicated (n = 3) (**C**) ANT-1 induces internucleosomal DNA cleavage. 293T cells were transfected with an empty vector or with an expression construct for ANT-1. Low molecular weight DNA was isolated from transfected cells, separated on a 2% agarose gel and stained with ethidium bromide.

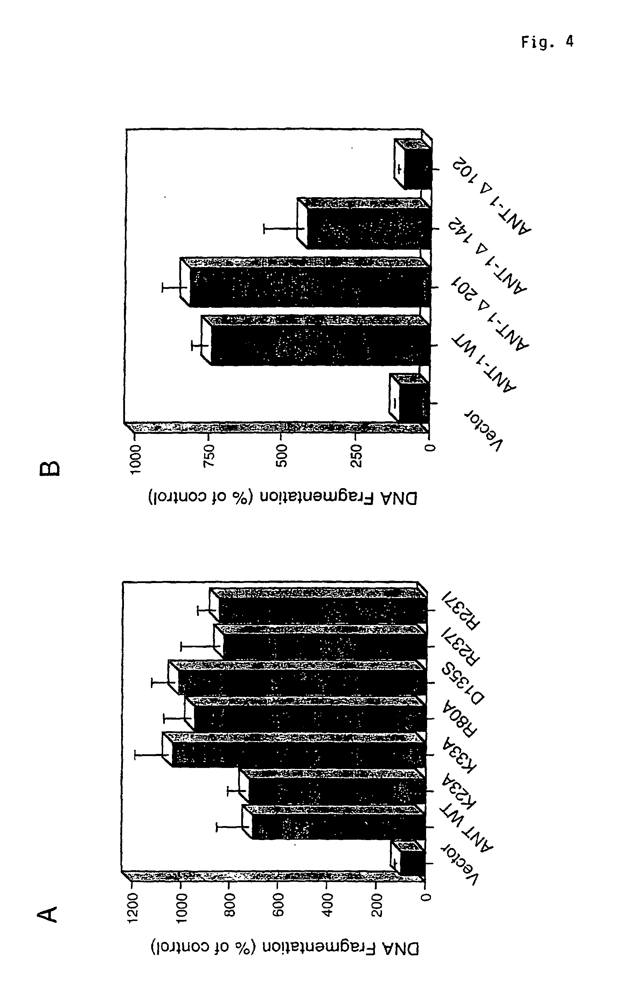

**Fig. 4**. Mutational analysis of ANT-1's apoptosis activity (**A**) Cell death induction by point mutants of ANT-1. Wild type ANT-1 (ANT-1 WT) and six point mutants that have been shown to be deficient for ADP/ATP transport were transfected into 293T cells. The WT amino acid, its position as well as the mutated residue are given for each mutant construct. After 16 hours, apoptosis induction by the various constructs was measured by an ELISA for internucleosomal DNA fragments. The DNA fragmentation as percentage of control is given as an index for apoptosis induction. The means and the standard deviations are indicated (n = 3) (B) Apoptosis activity of ANT-1 deletion mutants. Wild type ANT-1 (1 µg) and three C-terminal deletion mutants (1 µg) were transiently transfected into 293T cells. The last C-terminal residue of each deletion mutant is indicated. After 16 hours, the constructs were tested for apoptosis induction by a specific ELISA as described in A.

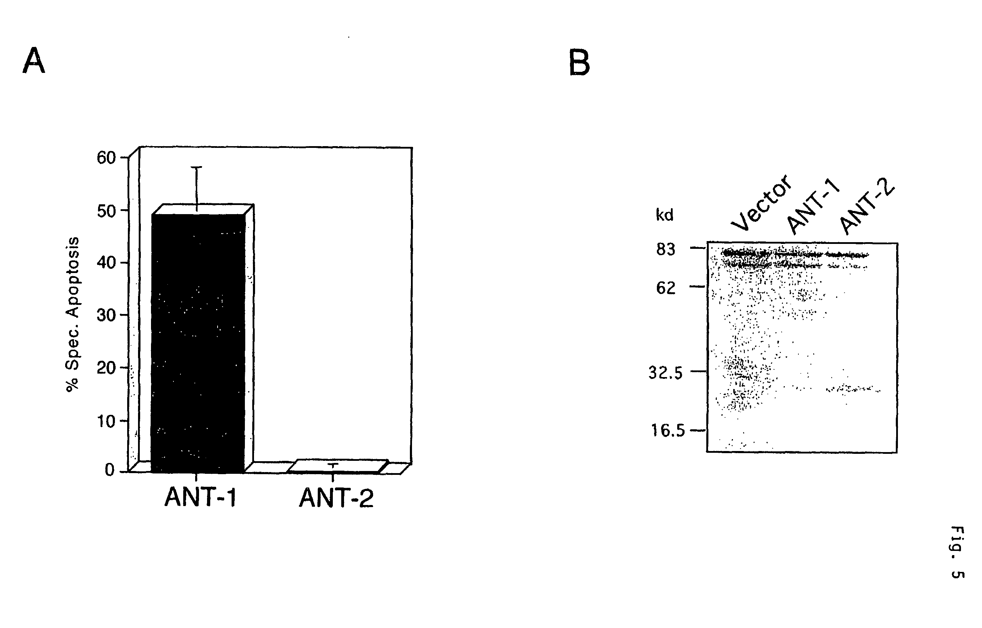

**Fig. 5.** Effect of ANT-1 and ANT-2 on transfected cells. (**A**) Comparison between the apoptosis capacities of ANT-1 and ANT-2. Expression plasmids for ANT-1 (1 µg) and ANT-2 (1 µg) were transfected in 293T cells. After 16 hours apoptosis induction in transfected cells was measured by FACS analysis of sub-G1 positive cells. The specific apoptosis induction above background is shown as percentage of apoptotic cells relative to all transfected cells. The means and the standard deviations are indicated (n = 3). (**B**) Expression and localization of ANT-1 and ANT-2. 293T cells were transfected with a control vector or expression vectors for HA-tagged ANT-1 and ANT-2. Mitochondrial extracts of transfected cells were prepared and investigated for the presence of the proteins with an anti-HA antibody in a western blot. Equal loading of the gel was verified by two unspecific upper bands.

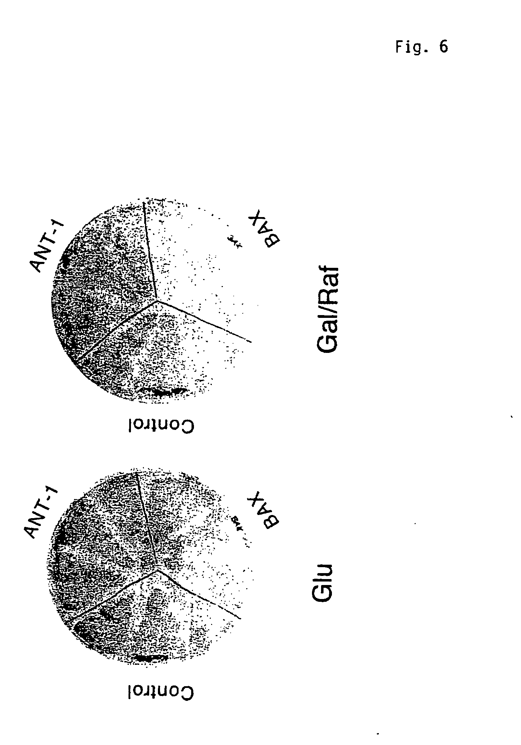

**Fig. 6.** Effect of Bax and ANT-1 expression on the growth of yeast cells. Bax and ANT-1 cloned in expression vectors under the control of a galactose and raffinose-inducible promoter and the empty control vector were introduced into yeast cells. 4,5x10⁴ yeast cells of three independent clones each were plated on glucose (glu)- or galactose and raffinose (gal/raf)-containing agar. The growth of the yeast cells was monitored 36 hours later.

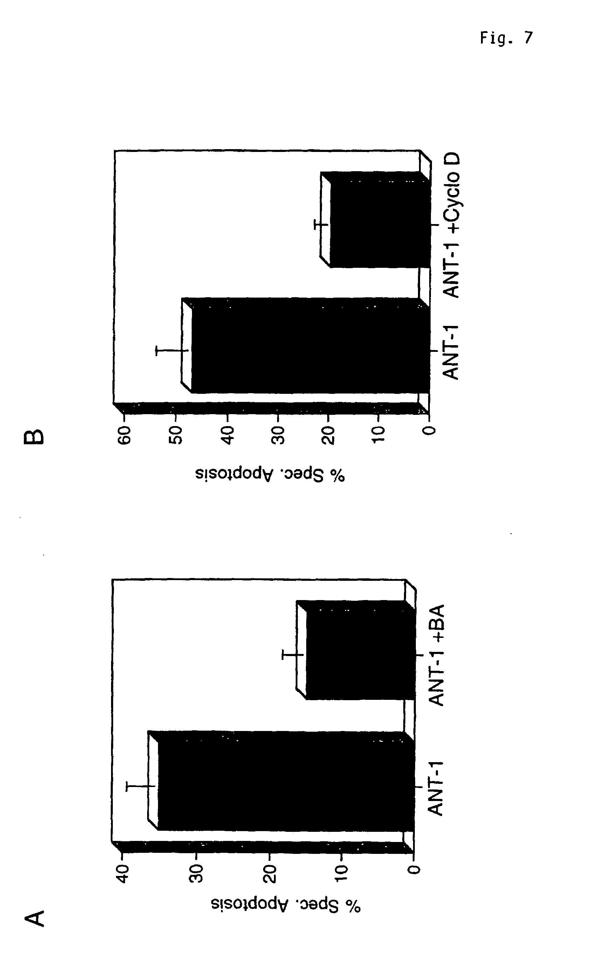

**Fig. 7**. Inhibition of ANT-1 induced apoptosis. (**A**) ANT-1-induced apoptosis can be repressed by bongkrekic acid, a specific inhibitor of the PT pore. An expression vector for ANT-1 (1 µg) was transfected into plates with 293T cells. One group of plates was left untreated, the other was supplied with 50 µM bongkrekic acid ("BA") for 16 hours after the transfection. Apoptosis was quantified as in figure 5. (**B**) ANT-1 apoptosis can be repressed by cyclophilin D, another component of the permeability transition pore. ANT-1 (1 µg) and an expression plasmid (10 µg) for cyclophilin D ("Cyclo D") or a control vector (10 µg) were transfected into 293T cells, and apoptosis was determined as described in figure 5.

### Examples

### 1.Materials and methods

### 1.1 Plasmids

The point mutants of ANT-1 were engineered by recombinant PCR using suitable primers. For the PCR reaction, Pwo, a thermostable polymerase with proof-reading function (Roche Diagnostics, Mannheim, Germany) was employed. All amino acid changes were verified by sequencing. Deletion mutagenesis of ANT-1 was achieved with PCR using the same enzyme. ANT-2 was amplified from a mouse kidney cDNA library, cyclophilin D from a 293T library, both by using specific primers. The correct sequence was verified by sequencing. The eukaryotic expression vector for baculovirus p35 has been described (Clem and Miller, 1994).

### 1.2 lmmuno Blotting

For detecting cytochrome c, 293T cells were transfected with an expression vector for ANT-1. After 18 hours the cells were harvested and cytoplasmic extracts isolated as described (Ferrari et al., 1998). Protein samples of 30 µg were loaded on SDS-polyacrylamide gels (10%) and then electrophoretically transferred onto PVDF-membranes (Amersham, Braunschweig, Germany). Western blots were probed with a monoclonal cytochrome c antibody (Pharmingen, San Diego, CA) and anti-mouse-horseradish-peroxidase conjugated antibody prior to ECL-based detection (Amersham). For the poly-ADP-ribose polymerase (PARP) western blot, nuclear fractions were obtained by differential centrifugation as described previously (Schreiber et al., 1989). Aliquots of 50 µg protein were subjected to SDS-PAGE (8% polyacrylamide) and blotted onto PVDF-membranes. PARP and cleavage products were detected using a polyclonal serum directed against full length PARP (Roche Diagnostics), anti-rabbit-horseradish-peroxidase conjugated antibody (Amersham, Braunschweig) and the ECL-system. For the detection of HA-tagged ANT-isoforms, mitochondria were isolated by differential centrifugation as described previously (Brustovetsky and Klingenberg, 1996). 30 µg protein samples were separated on 10% polyacrylamide gels and blotted onto PVDF-membranes. Detection was performed using a mouse monoclonal antibody raised against the influenza virus HA-peptide (Roche Diagnostics, Mannheim, Germany) and the ECL-system (Amersham, Braunschweig).

### 1.3 Yeast methods

Standard yeast methods were applied (Ausubel et al., 1991). For the inducible expression of Bax and ANT-1, the cDNAs were subcloned into pYESTrp2 (Invitrogen) in which the B42-fusion moiety was removed.

### 1.4 Apoptosis detection

Low molecular weight DNA from apoptotic cells was isolated and detected as described (Grimm and Leder, 1997). Apoptosis induction was measured by an ELISA (Roche Diagnostics) that is specific for nucleosomal DNA fragments that are released during apoptosis. The recommendations of the manufacturer were followed. Equal transfection efficiencies were monitored by cotransfecting an expression plasmid for GFP. Other apoptosis quantifications were performed by flow cytometry (Bitzer et al., 1999). A cotransfected GFP expression plasmid was used to assess the transfection efficiency. The apoptotic population was put in relation to the percentage of GFP-positive cells. The apoptotic background of the vector-control was subtracted to obtain the specific apoptosis induction. Each condition was tested in at least three independent experiments.

### 1.5 Mitochondrial membrane gradient detection

After transfecting an expression vector for ANT-1 together with a plasmid for GFP, cells were loaded with Mitotracker RedCMXRos (Molecular probes, OR, USA) according to the suggestions of the manufacturer. Images were documented using a Zeiss Axioplan fluorescence microscope (Zeiss Oberkochen, Germany). RedCMXRos fluorescence was excited at 546 nm and emission was imaged at > 590 nm. GFP fluorescence was excited at 450-490 nm and emission was monitored at 515-565 nm.

### 2.Results

### 2.1 ANT-1 induces cell death

Using our screen for dominant, apoptosis-inducing genes, we isolated a cDNA whose expression elicited an especially fast and strong apoptosis response in cells. Sequencing revealed that the gene encoded ANT-1, the ADP/ATP translocator protein of the PT pore (Marzo et al., 1998b). ANT was active in a wide variety of cell types: every cell line tested so far underwent apoptosis after stimulation by ANT-1 (not shown). Figure 1 shows the phenotype of control- and ANT-1-transfected HEK 293T cells: whereas the vector-transfected cells displayed a normal morphology, ANT-1 expressing cells showed a constricted cytoplasm and blebbing of the plasma membrane.

Since ANT-1 is a component of the PT pore whose activation leads to a dissipation of the proton gradient Δψₘ over the inner mitochondrial membrane (Marzo et al., 1998b), we tested ANT-1 transfected cells for this membrane gradient. To this end, we cotransfected ANT-1 and an expression vector for the green fluorescent protein (GFP) into HeLa cells. The membrane gradient in transfected and therefore GFP-positive cells was assayed with CMXRos, a dye whose fluorescence is dependent on an intact membrane potential. Figure 2A shows that ANT-1 expressing cells displayed a reduced mitochondrial fluorescence with this dye. This differential staining is a very early feature of ANT-1-mediated apoptosis induction since it could be observed in cells which did not yet display an altered phenotype.
One of the consequences of mitochondrial damage is the release of cytochrome c from the inner mitochondrial membrane space (Kluck et al., 1997; Yang et al., 1997). Therefore, we assayed cytoplasm of ANT-1 transfected cells for the presence of cytochrome c. Using a western blot, we could detect this protein in the cytoplasmic fraction of ANT-1 transfected cells (figure 2B). The released cytochrome c can bind Apaf-1, a mammalian homolog of the C. elegans gene ced-4 which leads to the aggregation and activation of downstream caspases (Srinivasula et al., 1998). We therefore investigated the status of poly-ADP-ribose polymerase (PARP), one of the best-known caspase substrates, in ANT-1 transfected cells. A western blot for PARP (figure 3A) revealed that ANT-1 transfected cells degraded PARP and generated the expected protein fragments. Since we have also observed apoptosis induction in MCF-7 cells (data not shown), a cell line that does not express caspase-3 (Janicke et al., 1998), we conclude that ANT-1-induced apoptosis is not dependent on this particular isoenzyme.

Bax, a member of the Bel-2 gene family, has recently been shown to interact directly with ANT-1 (Marzo et al., 1998a) and is known to induce apoptosis (Oltvai et al., 1993). However, caspase activation does not seem to be a necessary event for Bax-induced cell death (Xiang et al., 1996). Therefore, we wanted to know whether caspase activation is required for ANT-1 apoptosis induction. To address this, we cotransfected ANT-1 and an expression vector for p35, a broad-range caspase inhibitor from baculovirus (Clem and Miller. 1994). A quantitative apoptosis assay revealed a 6-fold reduction in apoptosis induction when p35 was cotransfected (figure 3B). Another substrate for caspases is ICAD, an inhibitor of the DNase CAD (Sakahira et al., 1998). Its degradation leads to the activation of CAD and the internucleosomal degradation of the DNA, a well-known biochemical sign of apoptosis. To investigate the status of the DNA, we transfected 293T cells with a control vector or ANT-1 and isolated the low molecular weight DNA. Only ANT-1 transfected cells displayed the typical DNA ladder (figure 3C). These results show that ANT-1 leads to all phenotypic and biochemical alterations associated with apoptosis.

### 2.2 The N-terminal half of ANT-1 is sufficient for apoptosis induction

ANT-1 was originally described as an ADP/ATP transporter (Riccio et al., 1975). We therefore theorized that overexpressed ANT-1 leads to an increased ADP/ATP exchange which results in a net-import of positive charges over the inner mitochondrial membrane, the subsequent collapse of the membrane potential and apoptosis induction. To assess whether the transporter activity of ANT-1 is required for its apoptosis induction, we generated six different point mutations all of which have been shown to impede ANT-1's activity to transport ADP and ATP (Muller et al., 1996; Muller et al., 1997). However, upon transfection into 293T cells we have not observed any difference relative to the wildtype (WT) in their potential to induce apoptosis as measured by the release of internucleosomal DNA fragments (figure 4A). ANT-1 is a member of the mitochondrial carrier family which consists of proteins with three 100 residue repeats in which alternating regions of hydrophilic and hydrophobic residues exist. As integral membrane proteins they span the membrane six times, interrupted by short amphipathic helices that are likely to form the transport active moiety (Klingenberg and Nelson, 1994). To map the domains responsible for apoptosis induction, we have created various deletion mutants of ANT-1:ANT-1Δ 217 lacks the last transmembrane domain and the last amphipathic helix, ANT-1Δ 142 cuts the molecule in half and comprises only the first two transmembrane domains and the first amphipathic helix. ANT-1Δ 102 contains only the first membrane spanning domain. Figure 4B demonstrates that ANT-1Δ 217 is as efficient for apoptosis induction as WT ANT-1, whereas ANT-1Δ 142 revealed a 50% reduction of its apoptosis potential. The largest deletion, ANT-1Δ 102, was inactive for apoptosis induction.

### 2.3 ANT-2 is unable to induce apoptosis

The PT pore complex is supposed to function by the conversion of the specific ADP/ATP exchange transporter into an unselective pore (Zoratti and Szabo, 1995). However, since over half of the ANT-1 protein is dispensable for apoptosis induction, we wanted to know whether ANT-1 induces apoptosis by forming an unspecific pore on its own. We therefore assessed the specificity of apoptosis induction by ANT-1. To this end we made use of ANT-2, an over 90% identical gene to ANT-1 that is likewise ubiquitously expressed (Doerner et al., 1997). Transfection of ANT-2 did not lead to apoptosis induction as measured by FACS analysis (figure 5A). The correct mitochondrial localization and efficient expression of ANT-2 was verified by western blot (figure 5B).

### 2.4 Growth of yeast cells is not affected by ANT-1

Bax, a member of the Bcl-2 gene family, can induce a form of cell death by overexpression in yeast cells (Greenhalf et al., 1996; Jurgensmeier et al., 1997; Zha et al., 1996), probably by activating ANT-1 and the PT pore (Greenhalf et al., 1996; Marzo et al., 1998a). We therefore wanted to determine whether ANT-1 has the same capacity. To address this, we subcloned ANT-1 and Bax into a galactose and raffinose inducible yeast vector. The induction of these genes on suitable agar plates revealed that Bax led to a reduction in the growth of these cells, indicating cell death. In contrast, ANT-1 expressing yeast cells were completely unaffected and grew as well as on the control plate (figure 6).

### 2.5 Cyclophilin D can repress apoptosis induced by ANT-1

Our experiments with ANT-2 and cell death in yeast suggested that ANT-1's apoptotic activity is mediated by specific protein-protein interactions rather than by forming an active PT pore on its own. To check whether ANT-1 expression nevertheless activates the endogenous PT pore, we made use of bongkrekic acid, a specific inhibitor for the PT pore (Klingenberg et al., 1970). 50 µM of this compound repressed ANT-1 induced apoptosis in a quantitative apoptosis assay more than 2-fold (figure 7A). We therefore speculated that ANT-1 might titrate out some components of the endogenous PT pore and that replenishing these proteins might repress apoptosis. Consequently, we cotransfected ANT-1 and an expression vector for cyclophilin D, a component of the PT pore on the matrix side which directly interacts with ANT-1 (Woodfield et al., 1998). Quantification of apoptosis induction revealed a more than 2-fold reduced cell death by ANT-1 when cyclophilin D was cotransfected (figure 7B).

### References

Ausubel et al., 1991. Current Protocols in Molecular Biology, Wiley interscience, New York.
Bitzer et al., 1999. J.Virol. 73: 702-708.
Brustovetsky and Klingenberg, 1996. Biochemistry. 35:8483-8488.
Clem and Miller, 1994. Mol.Cell.Biol. 14: 5212-5222.
Dec and Fuster, 1994. N.Engl.J.Med. 331: 1564-1575.
Doerner et al., 1997. FEBS Lett. 414: 258-262.
Doerner et al., 1997. Mol.Cell Biochem. 174: 261-269.
Ferrari et al., 1998. J.Exp.Med. 188: 979-984.
Fulda et al., 1998. J.Biol.Chem. 273: 33942-33948.
Greenhalf et al., 1996. FEBS Lett. 380: 169-175.
Grimm and Leder, 1997. J. Exp. Med. 185: 1137-1142.
Janicke et al., 1998. J.Biol.Chem. 273: 9357-9360.
Jurgensmeier et al., 1997. Mol.Biol.Cell.8: 325-339.
Klingenberg, 1993. J.Bioenerg.Biomembr.25: 447-457.
Klingenberg et al., 1970. Biochem.Biophys.Res.Commun. 39: 344-351.
Klingenberg and Nelson, 1994. Biochim. Biophys. Acta. 1187:241-244. Kluck et al., 1997. Science. 275: 1132-1136.
Korsmeyer, 1995. Trends Genet. 11: 101-105.
Loo et al., 1993. Proc.Natl.Acad.Sci. U S A. 90: 7951-7955.
Marzo et al., 1998a. Science. 281: 2027-2031.
Marzo et al., 1998b. J. Exp. Med. 187: 1261-1271.
McCarthy and Dixit, 1998. J.Biol.Chem. 273: 24009-24015.
McGill, G. 1997. Front.Biosci. 2: d353-379.
Muller et al., 1996. Biochemistry. 35: 16132-16143.
Muller et al., 1997. Biochemistry. 36: 16008-16018.
Narula et al., 1996. N.Engl.J.Med. 335: 1182-1189.
Newmeyer et al., 1994. Cell. 79: 353-364.
Oltvai et al., 1993. Cell. 74: 609-619.
Petit et al., 1995. J.Cell.Biol,130: 157-167.
Riccio et al., 1975. FEBS Lett. 56: 133-138.
Ruck et al., 1998. FEBS Lett.426: 97-101.
Sakahira et al., 1998. Nature. 391: 96-99.
Salvesen, 1997. Cell. 91: 443-446.
Schreiber et al., 1989. Nucleic Acids Res. 17: 6419.
Srinivasula et al., Mol. Cell. 1: 949-957.
Susin et al., 1999a. J. Exp. Med. 189: 381-394.
Susin et al., 1999b. Nature. 397: 441-446.
Vander Heiden et al., 1997. Cell. 91: 627-637.
White, 1996. Genes Dev. 10: 1-15.
Woodfield et al., 1998. Biochem J. 336: 287-290.
Xiang et al., 1996. Proc.Natl.Acad.Sci. U S A. 93: 14559-14563.
Yamamura et al., 1999. Jpn.Circ.J.63: 149-154.
Yang et al., 1997. Science. 275: 1129-1132.
Yang et al., 1998. Science. 281: 1355-1357.
Yao et al., 1996. J.Mol.Cell.Cardiol. 28: 95-101.
Zamzami et al., 1995a. J.Exp.Med. 182: 367-377.
Zamzami et al., 1995b. J.Exp.Med.181: 1661-1672.
Zamzami et al., 1998. Oncogene. 16: 1055-1063.
Zha et al., 1996. Mol.Cell.Biol. 16: 6494-6508
Zoratti and Szabo, 1995. Biochim.Biophys.Acta. 1241: 139-176.

## Claims

1. The use of an effective amount of a substance capable of inhibiting the activity of adenine nucleotide translocase-1 (ANT-1) for the inhibition of apoptosis in an isolated cell.

2. Use of claim 1, wherein said cell is a mammalian cell.

3. Use of claim 2, wherein said cell is associated with a pathogenic disorder.

4. Use of claim 1, wherein the activity of ANT-1 is inhibited on the nucleic acid level.

5. Use of claim 4, wherein the inhibition is effected by reducing ANT-1 gene expression.

6. Use of claim 4, wherein the activity of the endogenous ANT-1 promoter is reduced.

7. Use of claim 1, wherein the activity of ANT-1 is inhibited on the protein level.

8. Use of claim 7, wherein the inhibtion is effected by adding ANT-1 protein antagonists.

9. Use of claim 8, wherein the antagonist is cyclophilin D.

10. Use of claim 1, wherein an apoptosis-inducing signal transduction pathway is inhibited, said pathway being activated by ANT-1.

11. Use of a pharmaceutically effective amount of a substance capable of inhibiting the activity of adenine nucleotide translocase (ANT-1) for the manufacture of a medicament for the treatment of degenerative diseases associated with excessive apoptosis.

12. Use of claim 11, wherein the disease is a human disease.

13. Use of claims 11 or 12, wherein the disease is dilated cardiomyopathy.

14. Use of any one of claims 11 to 13, wherein the medicament is a pharmaceutical composition comprising pharmaceutically acceptable diluents, carriers or adjuvants.

15. An in vitro method for identifying substances suitable for apoptosis inhibition comprising the step of determining the capability of a test substance to inhibit the activity of ANT-1 by determining the capability of the test substance
(i) to bind ANT-1 or a domain thereof, or
(ii) to inhibit the binding of ANT-1 to natural binding partners thereof,
wherein a substance which binds ANT-1 or a domain thereof or inhibits the binding of ANT-1 to natural binding partners thereof is identified as an apoptosis inhibitor.

16. The method of claim 15, wherein the capability of a test substance to bind the N-terminal domain of ANT-1 is determined.

17. The method of claim 15, which is carried out as a high-throughput assay.

18. The method of claim 17, comprising a parallel determination of at least 96 test compounds.

19. The method of claim 15, which is carried out as a cell-based assay.

20. The method of claim 19, which is carried out as an assay using ANT-1-containing cell fractions or ANT-1-containing whole cells.

21. The method of claim 15, which is carried out as a molecular-based assay using an isolated protein selected from ANT-1, or a domain thereof.

22. The method of claim 21, wherein a recombinant protein is used.

23. The method of claim 15, wherein the determining step comprises the measurement of apoptosis induction.

24. The method of claim 23, wherein the apoptosis induction is measured by a parameter selected from the group consisting of DNA fragmentation, caspase activation or characteristic alterations in cell morphology.

25. An in vitro method for the diagnosis of an apoptotic process in a degenerative disease or a predisposition therefor comprising detecting the ANT-1 expression in a sample from the tissue and/or body fluids of a subject to be tested, wherein elevated ANT-1 expression is indicative for an apoptotic process occuring in a degenerative disease or a predisposition therefor.

26. The method of claim 25, wherein the degenerative disease is dilated cardiomyopathy.

27. Use of a pharmaceutical composition comprising as an active agent cyclophilin D for the manufacture of a medicament for the treatment of degenerative diseases associated with apoptosis.

## Patentansprüche

1. Verwendung einer wirksamen Menge einer Substanz, die in der Lage ist, die Aktivität von Adenin-Nukleotid-Translocase-1 (ANT-1) zu hemmen, zur Hemmung von Apoptose in einer isolierten Zelle.

2. Verwendung nach Anspruch 1, wobei die Zelle eine Säugerzelle ist.

3. Verwendung nach Anspruch 2, wobei die Zelle mit einer pathogenen Erkrankung im Zusammenhang steht.

4. Verwendung nach Anspruch 1, wobei die Aktivität von ANT-1 auf der Nukleinsäureebene gehemmt wird.

5. Verwendung nach Anspruch 4, wobei die Aktivität durch Verringerung der ANT-1 Genexpression bewirkt wird.

6. Verwendung nach Anspruch 4, wobei die Aktivität des endogenen ANT-1 Promotors verringert wird.

7. Verwendung nach Anspruch 1, wobei die Aktivität von ANT-1 auf der Proteinebene gehemmt wird.

8. Verwendung nach Anspruch 7, wobei die Hemmung durch Zugabe von ANT-1 Proteinantagonisten bewirkt wird.

9. Verwendung nach Anspruch 8, wobei der Antagonist Cyclophilin D ist.

10. Verwendung nach Anspruch 1, wobei ein Apoptose-induzierender Signalübertragungsweg gehemmt wird, wobei der Weg durch ANT-1 aktiviert wird.

11. Verwendung einer pharmazeutisch wirksamen Menge einer Substanz, die in der Lage ist, die Aktivität von Adenin-Nukleotid-Translocase-1 (ANT-1) zu hemmen, zur Herstellung eines Medikaments für die Behandlung von degenerativen Erkrankungen die mit übermäßiger Apoptose im Zusammenhang stehen.

12. Verwendung nach Anspruch 11, wobei die Erkrankung eine menschliche Erkrankung ist.

13. Verwendung nach Anspruch 11 oder 12, wobei die Erkrankung dilatative Kardiomyopathie ist.

14. Verwendung nach einerm der Ansprüche 11 bis 13, wobei das Medikament eine pharmazeutische Zusammensetzung ist, die pharmazeutisch annehmbare Verdünnungsmittel, Träger oder Hilfsstoffe umfasst.

15. In-vitro Verfahren zum Identifizieren von Substanzen, die zur Apoptosehemmung geeignet sind, umfassend den Schritt des Bestimmens der Fähigkeit einer Testsubstanz, die Aktivität von ANT-1 zu hemmen, durch Bestimmen der Fähigkeit der Testsubstanz
(i) ANT-1 oder eine Domäne davon zu binden, oder
(ii) die Bindung von ANT-1 an deren natürliche Bindungspartner zu hemmen,
wobei eine Substanz, die ANT-1 oder eine Domäne davon bindet oder die Bindung von ANT-1 an deren natürliche Bindungspartner hemmt, als Apoptoseinhibitor identifiziert wird.

16. Verfahren nach Anspruch 15, wobei die Fähigkeit einer Testsubstanz, die N-terminale Domäne von ANT-1 zu binden, bestimmt wird.

17. Verfahren nach Anspruch 15, welches als Hochdurchsatz-Assay durchgeführt wird.

18. Verfahren nach Anspruch 17, welches eine parallele Bestimmung von wenigstens 96 Testverbindungen umfasst.

19. Verfahren nach Anspruch 15, welches als ein Assay auf Zellbasis durchgeführt wird.

20. Verfahren nach Anspruch 19, welches als ein Assay unter Verwendung von ANT-1-haltigen Zellfraktionen oder vollständigen ANT-1-haltigen Zellen durchgeführt wird.

21. Verfahren nach Anspruch 15, welches als ein Assay auf molekularer Basis unter Verwendung eines isolierten Proteins ausgewählt aus ANT-1 oder einer Domäne davon, durchgeführt wird.

22. Verfahren nach Anspruch 21, wobei ein rekombinantes Protein verwendet wird.

23. Verfahren nach Anspruch 15, wobei der Bestimmungsschritt die Messung der Apoptoseinduzierung umfasst.

24. Verfahren nach Anspruch 23, wobei die Apoptoseinduzierung durch einen Parameter gemessen wird, der ausgewählt ist aus der Gruppe bestehend aus DNA-Fragmentierung, Caspaseaktivierung oder charakteristischen Veränderungen der Zellmorphologie.

25. In-vitro Verfahren zur Diagnose eines apoptotischen Vorgangs bei einer degenerativen Erkrankung oder einer Prädisposition dafür, umfassend Detektion der ANT-1 Expression in einer Gewebeprobe und/oder Körperflüssigkeitsprobe eines zu untersuchenden Subjekts, wobei eine erhöhte ANT-1 Expression ein Anzeichen für einen apoptotischen Vorgang ist, der bei einer degenerativen Erkrankung oder einer Prädisposition dafür auftritt.

26. Verfahren nach Anspruch 25, wobei die degenerative Erkrankung dilatative Kardiomyopathie ist.

27. Verwendung einer pharmazeutischen Zusammensetzung, die als einen Wirkstoff Cyklophilin D umfasst, zur Herstellung eines Medikaments zur Behandlung von degenerativen Erkrankungen, die mit Apoptose im Zusammenhang stehen.

## Revendications

1. Utilisation d'une quantité efficace d'une substance capable d'inhiber l'activité adénine-nucléotide-translocase-1 (ANT-1) pour inhiber l'apoptose dans une cellule isolée.

2. Utilisation selon la revendication 1, dans laquelle ladite cellule est une cellule de mammifère.

3. Utilisation selon la revendication 2, dans laquelle ladite cellule est associée à un trouble pathogène.

4. Utilisation selon la revendication 1, dans laquelle l'activité de ANT-1 est inhibée au niveau acide nucléique.

5. Utilisation selon la revendication 4, dans laquelle l'inhibition s'effectue par réduction de l'expression génique de ANT-1.

6. Utilisation selon la revendication 4, dans laquelle l'activité du promoteur endogène de ANT-1 est réduite.

7. Utilisation selon la revendication 1, dans laquelle l'activité de ANT-1 est inhibée au niveau protéique.

8. Utilisation selon la revendication 7, dans laquelle l'inhibition s'effectue par addition d'antagonistes de la protéine ANT-1.

9. Utilisation selon la revendication 8, dans laquelle l'antagoniste est la cyclophiline D.

10. Utilisation selon la revendication 1, dans laquelle une voie de transduction du signal induisant l'apoptose est inhibée, ladite voie étant activée par ANT-1.

11. Utilisation d'une quantité pharmaceutiquement efficace d'une substance capable d'inhiber l'activité adénine-nucléotide-translocase (ANT-1) pour la fabrication d'un médicament destiné au traitement de maladies dégénératives associées à une apoptose excessive.

12. Utilisation selon la revendication 11, dans laquelle la maladie est une maladie humaine.

13. Utilisation selon les revendications 11 ou 12, dans laquelle la maladie est la cardiomyopathie dilatée.

14. Utilisation selon l'une quelconque des revendications 11 à 13, dans laquelle le médicament est une composition pharmaceutique comprenant des diluants, supports ou adjuvants pharmaceutiquement acceptables.

15. Procédé d'identification *in vitro* de substances appropriées pour l'inhibition de l'apoptose, comprenant l'étape de détermination de la capacité d'une substance d'essai à inhiber l'activité de ANT-1 par la détermination de la capacité de la substance d'essai
(i) à se lier à ANT-1 ou à l'un de ses domaines, ou
(ii) à inhiber la liaison de ANT-1 à ses partenaires de liaison naturels,
dans lequel une substance qui se lie à ANT-1 ou à l'un de ses domaines ou qui inhibe la liaison de ANT-1 à ses partenaires de liaison naturels est identifiée comme un inhibiteur de l'apoptose.

16. Procédé selon la revendication 15, dans lequel on détermine la capacité d'une substance d'essai à se lier au domaine N-terminal de ANT-1.

17. Procédé selon la revendication 15, qui est effectué sous forme d'une analyse à haut débit.

18. Procédé selon la revendication 17, comprenant une détermination parallèle d'au moins 96 composés d'essai.

19. Procédé selon la revendication 15, qui est effectué sous forme d'une analyse à base de cellules.

20. Procédé selon la revendication 19, qui est effectuée sous forme d'une analyse utilisant des fractions de cellules contenant de ANT-1 ou des cellules entières contenant ANT-1.

21. Procédé selon la revendication 15, qui est effectué sous forme d'une analyse à base moléculaire utilisant une protéine isolée choisie parmi ANT-1 ou un de ses domaines.

22. Procédé selon la revendication 21, dans lequel on utilise une protéine recombinante.

23. Procédé selon la revendication 15, dans lequel l'étape de détermination comprend la mesure de l'induction de l'apoptose.

24. Procédé selon la revendication 23, dans lequel l'induction de l'apoptose est mesurée par un paramètre choisi dans le groupe constitué par la fragmentation d'ADN, l'activation d'une caspase ou des altérations caractéristiques de la morphologie cellulaire.

25. Procédé *in vitro* de diagnostic d'un processus apoptotique dans une maladie dégénérative ou une prédisposition pour cette maladie, comprenant la détection de l'expression de ANT-1 dans un échantillon de tissu et/ou de fluides corporels d'un sujet à tester, dans lequel une expression élevée de ANT-1 indique l'existence d'un processus apoptotique dans une maladie dégénérative ou une prédisposition pour cette maladie.

26. Procédé selon la revendication 25, dans lequel la maladie dégénérative est la cardiomyopathie dilatée.

27. Utilisation d'une composition pharmaceutique comprenant comme agent actif de la cyclophiline D pour la fabrication d'un médicament destiné au traitement de maladies dégénératives associées à une apoptose.
